Europäisches Patentamt

(19) European Patent Office

Office européen des brevets

(11) Publication number: **0 028 601**
**B1**

(12) **EUROPEAN PATENT SPECIFICATION**

(45) Date of publication of patent specification: **31.08.83**

(51) Int. Cl.³: **F 16 L 37/28, F 16 L 29/00**

(21) Application number: **80900257.9**

(22) Date of filing: **22.01.80**

(86) International application number:
**PCT/SE80/00019**

(87) International publication number:
**WO 80/01507 24.07.80 Gazette 80/17**

(54) **SLIDE VALVE AND COUPLER ASSEMBLY.**

(30) Priority: **22.01.79 US 5427**
**06.02.79 SE 7901012**

(43) Date of publication of application:
**20.05.81 Bulletin 81/20**

(45) Publication of the grant of the patent:
**31.08.83 Bulletin 83/35**

(84) Designated Contracting States:
**AT DE FR GB NL SE**

(56) References cited:
**GB - A - 576 681**
**US - A - 2 687 903**
**US - A - 2 709 090**
**US - A - 2 757 941**
**US - A - 2 779 608**
**US - A - 2 799 517**

(73) Proprietor: **SVENSSON, Jan Axel**
**Solhemsgatan 12**
**S-561 35 Huskvarna (SE)**

(72) Inventor: **SVENSSON, Jan Axel**
**Solhemsgatan 12**
**S-561 35 Huskvarna (SE)**

(74) Representative: **Ström, Tore et al,**
**c/o Ström & Gulliksson AB Rundelsgatan 14**
**S-211 36 Malmö (SE)**

Courier Press, Leamington Spa, England.

## Slide valve and coupler assembly

This invention relates to a slide valve and coupler assembly for controlling fluid flow, comprising a slide valve including a valve housing having a first through fluid flow passage, at least one slide valve member, and guide means in the housing for guiding the slide valve member along said guide means between a closed position across and closing off said first passage to fluid flow and an open position leaving the passage open for fluid flow, and a coupler including a coupler housing having a second through fluid flow passage.

A slide valve and coupler assembly of this type is disclosed in US—A—2,779,608.

When body fluids are being drained or removed from the body or fluids such as medicaments are being administered to the body, it is frequently necessary to change the receptacle in which the fluid are being collected, when full, or the reservoir supplying the fluid or medicament, when exhausted. At such times it is important that the connection to the body be closed, as well as the receptacle, and it is also desirable that the change be made as quickly as possible without spillage or leakage of the fluid and without any risk of bacteria being transferred between the passage for the fluid and the exterior of the means used for establishing said connection.

The slide valve and coupler assembly of US—A—2,779,608 does not satisfy these requirements since the fluid flow passage of the coupler when disconnected from the slide valve is left open, no means being provided for closing off said fluid flow passage.

One field more closely studied by the inventor, in which the related considerations apply, is the collection of urine from incontinent patients in hospitals. For this purpose it is customary to use a drainage system including a receptacle bag of plastics material, which is suspended at the bed or is carried by the patient, a catheter, and a coupler assembly connecting the receptacle bag to the catheter or to a hose connected with the catheter. The bar is coupled to the catheter or hose in such a way that it can be easily disconnected therefrom when it is necessary to replace the bag; such replacement has to take place about 4 times each 24 hours. At present unprotected coupler assemblies are provided for the disengageable connection of the bag to the catheter or hose, i.e. when a bag is substantially full and shall be disconnected from the catheter or hose this is clamped upstream of the coupler assembly and the bag is then disconnected without being sealed at the inlet. Accordingly, urine is left at the exterior of the parts of the coupler assembly, associated with the bag and the catheter or hose, respectively, and as will be realized such residues of urine on the coupler assembly parts provide a great risk of bacteria growth and bacteria transfer when the bags are handled and patients carrying such bags are walking around within the hospital.

The same problems are encountered when the urine is collected in a plastics bag receptacle or other container having a closable bottom outlet, which is emptied periodically through said outlet.

The primary purpose of the invention is to provide a slide valve and coupler assembly for use in carrying body fluids, which allows connection and disconnection of fluid carrying parts in a completely closed way in order to prevent bacteria growth and to avoid bacteria transfer to the interior of the body and to the surroundings. By the prevention of bacteria transfer to the hospital environment, the transfer of infections between the patients, to the personnel and between different wards of the hospital is also prevented.

It is also the purpose of the invention to provide a coupler assembly of the kind referred to, which avoids leakage of body fluids when receptacles or reservoirs thereof are connected to and disconnected from a body drainage or administering system.

In accordance with the invention such leakage, bacterial transfer, and spillage are prevented by a slide valve and coupler assembly of the type referred to above, which is characterized in that said coupler comprises a first recess for receiving at least the slide valve member-containing portion of the slide valve housing therein with said first passage and said second passage in alignment, operating means which is adjustable in said coupler housing between a first position across and closing off said second passage, and a second position leaving said second passage open for fluid flow, at least one second recess in said operating means for receiving the slide valve member when the valve housing is inserted in the first recess, said operating means being adjustable for sliding the slide valve member to said closed position when the operating means is adjusted to said first position, and to said open position when the operating means is adjusted to said second position, and locking means on said operating means connecting with the valve housing when the operating means is operated to said second position.

Preferred embodiments of the slide valve and coupler assembly according to the invention are illustrated in the accompanying drawings, in which:

Fig. 1 is a perspective view of the slide valve and coupler assembly as used in connection with a catheter the slide valve and the coupler being shown in disconnected position,

Fig. 2 is a partly broken perspective view of the slide valve and coupler assembly of Fig. 1 in closed position, such as when the slide valve is first installed in the coupler housing,

Fig. 3 is a longitudinal cross-sectional view of the slide valve and coupler assembly of Figs. 1 and 2 in the closed position as shown in Fig. 2,

Fig. 4 is a partly broken perspective view as that in Fig. 2 but showing the slide valve and coupler assembly in the open position for fluid flow through the assembly,

Fig. 5 is an exploded perspective view of the slide valve,

Fig. 6 is a perspective view of the slide valve and coupler assembly as used in interconnecting a catheter hose and a receptacle for fluids drained by the catheter, the slide valve and coupler assembly being shown in the closed position with the slide valve disconnected from the coupler housing,

Fig. 7 is a perspective view of the slide valve and coupler assembly with a fluid measuring apparatus connected therewith,

Fig. 8 is a perspective view of an apparatus for emptying fluid receptacles as that shown in Fig. 6, said apparatus including a number of couplers according to the invention,

Fig. 9 is a perspective view of the slide valve and coupler assembly of the invention with a syringe connected to the slide valve,

Fig. 10 is a longitudinal cross-sectional view of another embodiment of the slide valve with a syringe shown in elevational side view, connected therewith,

Fig. 11 is a longitudinal cross-sectional view of a further embodiment of the slide valve and coupler assembly, the slide valve and the coupler being shown disconnected from each other,

Fig. 12 is a longitudinal cross-sectional view of a still further embodiment of the slide valve,

Fig. 13 is a longitudinal cross-sectional view of the slide valve and coupler assembly with the slide valve shown in Fig. 12 connected with the coupler,

Fig. 14 is a partly broken perspective view of a still further embodiment of the slide valve and coupler assembly shown in a first closed position,

Fig. 15 is a longitudinal cross-sectional view of the slide valve and coupler assembly as shown in Fig. 14,

Fig. 16 is a partly broken perspective view of the embodiment of Figs. 14 and 15 shown in the open position, and

Fig. 17 is a partly broken perspective view of the embodiment of Figs. 14 to 16 shown in a second closed position.

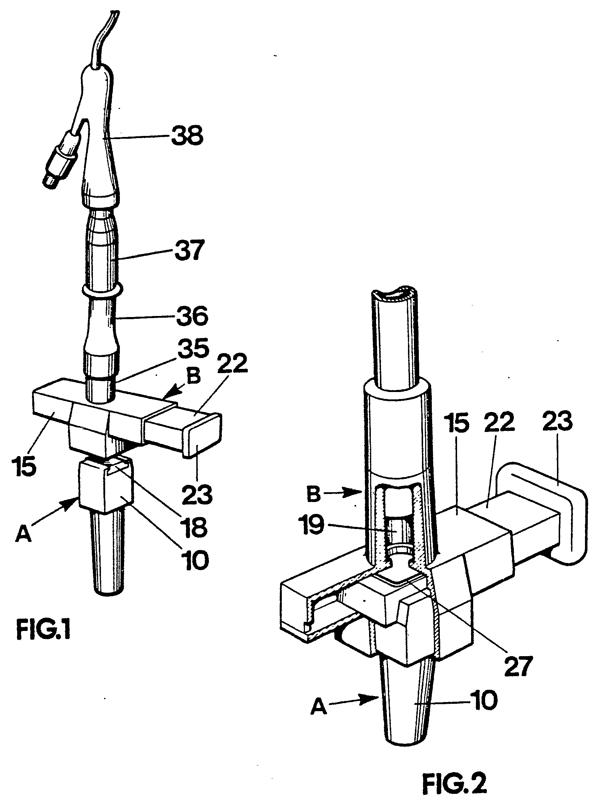

Referring to Figs. 1 to 5 in the drawings, the slide valve generally designated A, of the slide valve and coupler assembly has a valve housing 10 with a through fluid flow passage 11 forming a fluid inlet or outlet port 12 at one end thereof and a fluid outlet or inlet port 13 at the other end thereof. The valve housing is shaped to receive a piece of tubing in a snug leak-tight fit, by which the passage 11 can be connected to a receptacle or other appliance at the outlet or inlet 12. However, the valve housing 10 can also form part of a receptacle or other appliance or can simply end in the open air for manual discharge of urine, as shown in Figs. 1 to 5.

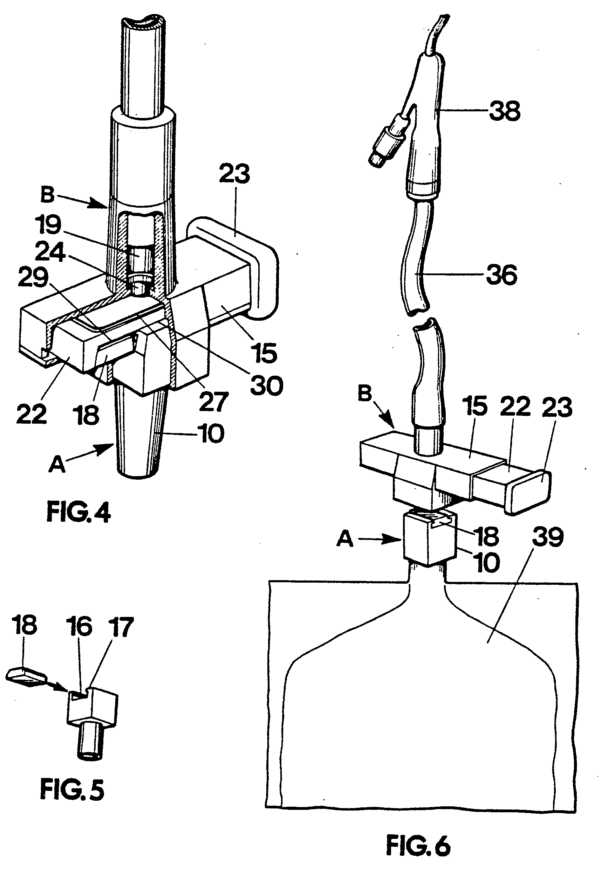

The valve housing 10 is shaped to fit within a recess 14 of a coupler housing 15 forming part of a coupler generally designated B. In the portion adjacent to the port 13 the housing is shaped with a reentrant slot 16, Fig. 5, having edge portions 17 and serving as a track for a slide valve member 18, along which the valve member can be slid between closed and open positions over the port 13 across and away from the trough passage 11. When the slide valve member 18 is in the position across the port 13 and passage 11, the passage is closed. When it is away from the port 13 and passage 11 the passage is open. In this position the valve member 18 may be out of the slot 18 entirely, whether or not the valve housing 10 is in position in the recess 14 of the coupler housing 15.

It will be noted that the valve member 18 has sides shaped to closely fit within the slot 16. The reentrant configuration of the slot retains the valve member 18 closely against the inner face of the housing 10, and a leak-tight seal at the port 13 is ensured by the valve member 18 being made of resilient rubber or rubber-like plastics material and engaging the bottom surface of the slot 16. However, the valve member 18 can also be made of a substantially rigid material, a sealing ring then being provided in the bottom of the groove 16 around the port 13 of the fluid flow passage 11.

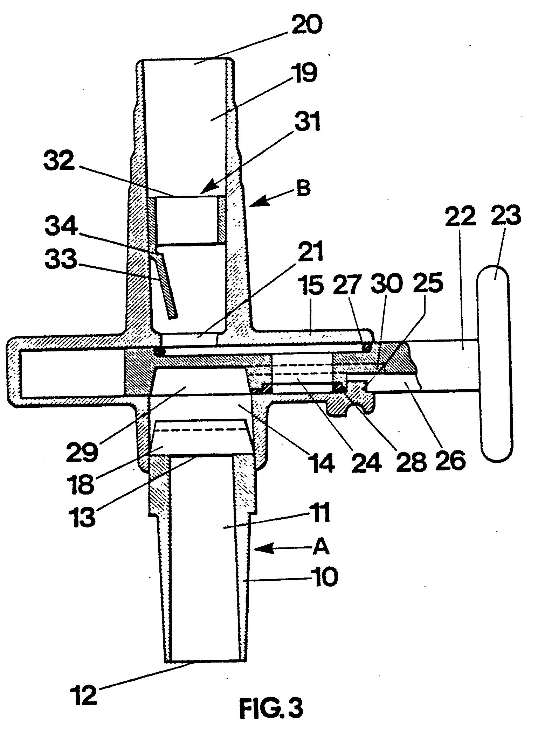

The coupler housing 15 has a through fluid flow passage 19 forming an inlet or outlet port 20 and an outlet or inlet port 21 at the ends thereof. The coupler housing is provided with an operating slide 22 forming a finger rest 23 at one end thereof. There is a through aperture 24 in the operating slide 22, and the slide is guided for displacement in the coupler housing 15 between a first position shown in Figs. 2 and 3 in which the aperture 24 is away from the port 21 and the coupler passage 19 accordingly is closed by the operating slide, and a second position, shown in Fig. 4, in which the aperture 24 is in register with the port 21 and the coupler passage 19 accordingly is open to fluid flow. Said first and second positions are determined by a protrusion 25 formed by the coupler housing 15 and engaging a longitudinal groove 26 in the operating slide 22.

The operating slide 22 is provided with a rectangular sealing ring 27 in the side facing the port 21, and this sealing ring surrounds said port and the aperture 24 in said first and second positions of the operating slide and every position therebetween. On the opposite side of the operating slide 22 facing the recess 14 a sealing element 28 is provided which surrounds the aperture 24.

The lower face of the operating slide 22 has a cut-out portion 29 shaped to receive the slide valve member 18 attached to the valve housing 10.

The slide valve A and the coupler B of the assembly of the invention are put together by placing the operating slide 22 in said first position shown in Figs. 2 and 3, and then inserting the slide valve into recess 14, the slide valve member 18 being received by the cut-out portion 29 in the operating slide 22. In this position of the slide valve in the coupler housing longitudinal side grooves 30 in the operating slide 22, opening into the cut-out portion 29, are in register with the edge portions 17 of the valve housing 10.

Accordingly, with the operating slide 22 in said first position shown in Figs. 2 and 3 and with the slide valve housing 10 inserted in the recess 14 in the coupler housing 15, the slide valve member 18 closing off the through passage 11 therein, the operating slide 22 can be displaced manually at the finger rest 23 to the left as seen in Fig. 3. By such displacement the slide valve 18 received by cut-out portion 29 will be displaced in the reentrant groove 16 to the left away from port 13, the aperture 24 at the same time being brought into register with the port 21 of the passage 19 in the coupler housing 15 and with the port 13 of the passage 11 in the slide valve housing 10. Thus, when the operating slide has arrived at the second position thereof, shown in Fig. 4, fluid flow communication is established through the slide valve and coupler assembly.

During the displacement of the operating slide 22 from the first position shown in Figs. 2 and 3 to the second position shown in Fig. 4 the edge portions 17 at the reentrant groove 16 in the slide valve housing 10 are received in the longitudinal side grooves in the operating slide 22, and by the engagement between the coupler and the slide valve housing thus provided the slide valve housing will be retained in the position shown in Fig. 4, received by the recess 14.

When the fluid flow connection thus provided is to be interrupted the operating slide 22 is returned to said first position shown in Figs. 2 and 3 manually pulling the operating slide at the finger rest 23 to the right as seen in Fig. 3. This closes off both the port 21 by the operating slide and the port 13 by the valve member 18 which is returned to the original closed position in the reentrant groove 16. At the same time the operating slide 22 is disengaged from the edge portions 17 at the side grooves 30. It is now possible to withdraw the slide valve housing 10 from the recess 14, carrying with it the slide valve member 18, and this operation can be performed without leakage or spillage.

The connection and disconnection of the slide valve to and from the coupler can be accomplished without contaminating a sterile environment, since no parts or surfaces of the assembly in contact with fluids passing therethrough will be exposed to the environment.

Also, no contamination will be transferred from the environment to the line or passage controlled by means of the assembly.

As disclosed in Fig. 3, a check valve 31 preventing back flow through the passage 19 can be provided in said passage. It comprises a bushing 32 of plastics material attached in the passage 19 by pressure fit or by other means such as adhesive, infrasound welding or the like. A closure member 33 is formed integrally with the bushing 32 and is connected therewith by a flexible web 34 allowing the closure member 33 to pivot between the open position shown in Fig. 3 and a closed position in which the closure member engages the lower annular end surface of the bushing and prevents a back flow through the bushing and thus through the passage 19.

Reverting to Fig. 1, the coupler housing 15 is connected at the portion 35 thereof through which the passage 19 extends, to a piece of tubing 36 such as a plastics hose in a snug leak-tight fit and this tubing is connected by a conventional adapter 37 to the outlet end coupling 38 of a catheter partly shown in Fig. 1, which is introduced in the usual manner into the urine bladder of a human being. Normally, the operating slide 22 of the slide valve and coupler assembly is in the closed position of Figs. 2 and 3 as is also shown in Fig. 1, and no slide valve A is connected to the coupler B. The urine thus will be collected in the urine bladder but will not be discharged therefrom until the operating slide 22 is manually displaced to the open position according to Fig. 4. Before this is done, a slide valve delivered in a sterile package is attached to the coupler housing in the manner described above, and the urine collected in the urine bladder is discharged in the water-closet through the passage 11 at the port 12. The discharge of urine can be performed in a fully satisfactory hygienic way. The slide valve when connected to the coupler housing is manually handled during the discharge of urine, which can be accomplished in an almost natural way. Then, the operating slide 22 is displaced to the closed position and the slide valve is disconnected from the coupler housing and thrown away.

It will accordingly be seen that the slide valve and coupler assembly can be used in this way for exercising the bladder to restore its flexibility.

In Fig. 6 the tubing 36 connecting the coupler housing 15 with the catheter coupling 38 can comprise a flexible rubber or plastics hose of considerable length. The slide valve housing 10 is permanently connected to or forms part of a conventional plastics bag receptacle 39 which may be suspended at the patient's bed or may be carried by the patient by suitable suspension means. Normally, the receptacle 39 is connected to the catheter coupling 38 by means of the slide valve and coupler assembly of the invention in the manner previously described, and when the receptacle is substantially full it can be replaced by an empty receptacle in the hygienic manner specific for the use of said assembly as described herein.

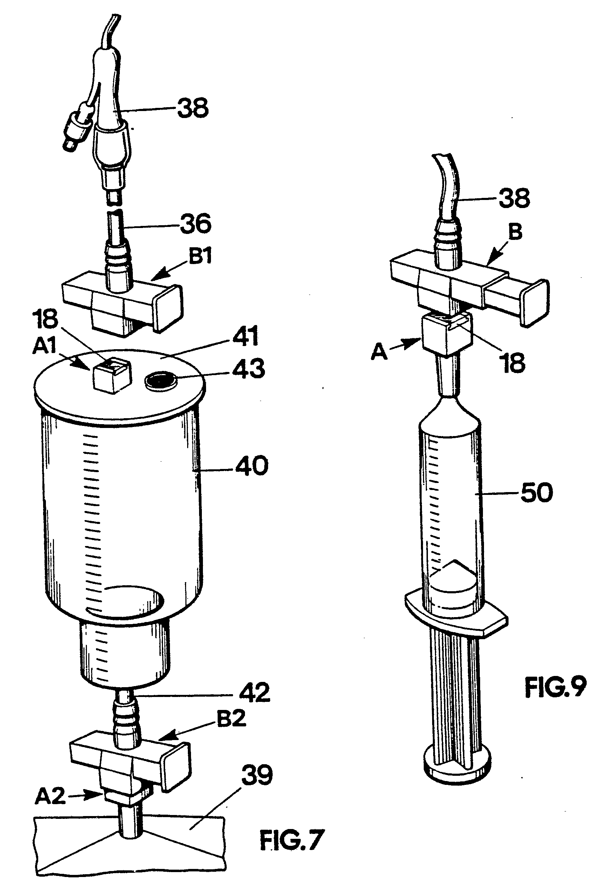

It is also possible to interconnect between the catheter coupling 38 and the receptacle 39 a urine measuring container 40 by using two slide valve and coupler assemblies of the invention as shown in Fig. 7. In that case, one assembly A1, B1 is used for coupling the catheter to the inlet in the lid 41 of the measuring container 40, said inlet being formed by the slide valve A1 of that assembly. The outlet 42 of the container 40 is connected to a second slide valve and coupler assembly A2, B2 for connecting the container 40 to the receptacle 39. When the catheter is connected to container 40 through slide valve A1 the outlet 43 is closed by means of the coupler B2 and the receptacle 39 is disconnected therefrom so that the urine will be continuously collected in the container 40, where the volume thereof can be measured. During the collection of the urine in the container this is vented through a sterile filter 43. From time to time the urine collected in the container 40 is discharged from the container into the receptacle 39 by connecting the receptacle to the container 40 at the assembly A2, B2.

When receptacles 39 of Figs. 6 and 7 have been filled with urine and have been disconnected from the coupler housing 15 they are completely closed off by means of the slide valve and therefore can be handled by the personnel in the hospital without the risk of bacteria being transferred therefrom, because the valve member 18 has been moved to the closing-off position by means of the operating slide 22 without being wetted by urine on the exterior sides thereof.

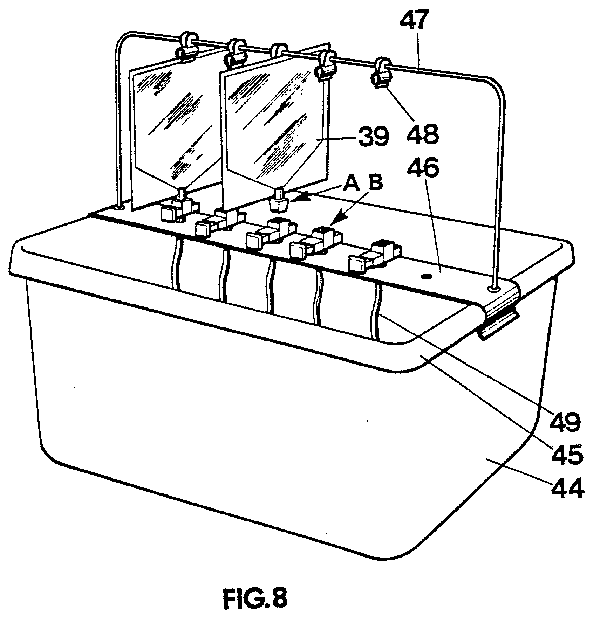

Fig. 8 discloses an apparatus for emptying receptacles 39. This apparatus comprises a tub 44 having a beaded rim 45, to which is attached a frame including a flat metal bar 46 extending between two opposite walls of the tub and engaging the rim thereof, and further including a wire 47 forming two vertical portions connected to the bar 46, and a horizontal portion extending in parallel with the bar spaced upwardly therefrom. On the wire 47 a plurality of spring-clips 48 are arranged for suspending the filled receptacles 39 turned upside down, and on the bar 46 a plurality of couplers A of the type described above are secured to the bar. The passage 19 of each coupler housing is connected to a tubing 49 opening into the tub 44. Thus, the slide valve A of each container 39 suspended in the apparatus can be connected to a coupler B for opening the slide valve and discharging the contents of the receptacle into the tub 44 in a hygiene manner. Tub 44 may be connected directly to a drainage or water-closet system.

The slide valve and coupler assembly according to the present invention advantageously can be used also in carrying and transferring other fluids than body fluids, e.g. sterilized fluids. Illustrative of this use of the assembly are flushing of the bladder and supply of sterile solution in peritoneal dialysis.

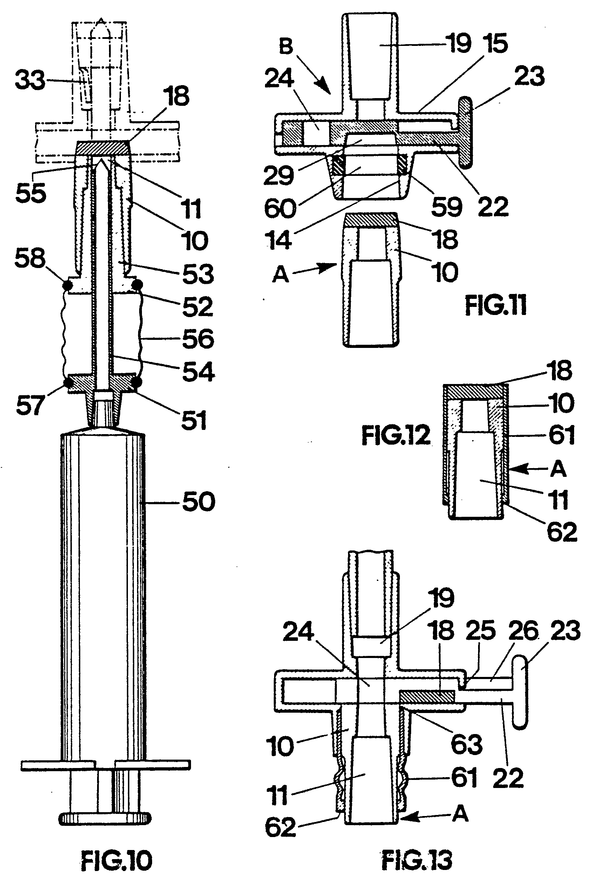

In Fig. 9 a syringe 50 of a conventional design is provided at the outlet thereof with the slide valve A forming part of the slide valve and coupler assembly of the invention. By means of the coupler B of the assembly, which is connected to a catheter coupling 38, the syringe 50 can be connected to the catheter for supplying a flushing liquid to the bladder by means of the syringe. This liquid can then be withdrawn again from the bladder by means of the syringe. Now, if a check valve, preventing back flow through the coupler B is arranged in the passage 19 as described with reference to Fig. 3, it is necessary to maintain this check valve opened, and this can be done by embedding a member of a magnetic metal in the closure member 33 such that said member can be operated to and retained in the open position by means of a permanent magnet approached to the outside of the coupler housing 18.

Fig. 10 discloses a mechanical arrangement for positively maintaining the check valve provided in the coupler housing 15 according to Fig. 3 in the opened position. In this case there is provided on the outlet of the syringe 50 an annular flange 51, and a corresponding flange 52 is provided on a connector piece 53 adapted to be connected with the slide valve housing 10. A tube 54 integral with the flange 51 and projecting therefrom extends displaceably through the connector piece 53 and terminates in a pointed end 55. A flexible bellows 56, e.g. of a thin latex material, is connected to the flanges 51 and 52 by means of clamp rings 57 and 58, respectively. When the slide valve has been connected to the coupler in the manner previously described and the valve member 18 has been displaced for opening the passage 11 through valve housing 10, the tube 54 can be axially displaced through the fluid flow passage extending through the open slide valve and coupler assembly so as to engage the closure member 33 and keep the closure member back so that it has no possibility to close. The flushing liquid supplied by means of the syringe 50 then can be delivered through the tube 54 to the passage 19 upstream of the check valve.

In order to further improve the safety against transfer of bacteria from the slide valve to the passage 19 of the coupler and the tubing or other conduit connected therewith the arrangement disclosed in Fig. 11 can be resorted to. More particularly, this embodiment provides improved safety against transfer of bacteria that may be located on the outside of the valve housing 10.

In Fig. 11 the operating slide is in the closed position when in the left hand position shown, and will be opened by pulling the operating slide to the right at the finger rest 23. This arrangement may be preferred for slide valve and coupler assemblies the normal position of which is the closed position, while the arrangement disclosed in Figs. 1 to 10 may be preferred for slide valve and coupler assemblies the normal position of which is the open position.

An inside annular groove 59 in the recess 14 formed by the coupler housing 15 receives a ring 60 of a porous or similar material in which a disinfection liquid is absorbed. The opening formed by the ring 60 the form of which corresponds to the cross-sectional form of the recess 14 is smaller than the cross section of the recess such that the ring projects inwardly from the inside surface of the recess. Accordingly, when the valve housing 10 is inserted into the recess 14 it will slide against the ring 60 and the outside surface of the valve housing will be wetted with the disinfection liquid. Moreover, the ring 60 will form a bacteria barrier in the recess when the valve housing 10 is inserted therein. Other disinfection means sliding against the outside of the valve housing may be arranged.

Another embodiment for the same purpose as the arrangement disclosed in Fig. 11 is disclosed in Figs. 12 and 13. A thin socket 61 of a flexible material such as latex is fixedly attached to the outside of the valve housing 10 at 62 and extends over said surface to the end face of the valve member 18. The recess 14 forms an inside shoulder 63, and when the valve housing 10 is inserted into the recess in order that the valve member shall be received in the cut-out portion 29 the socket 61 will engage the shoulder 63 and thus will be held back at continued displacement of the housing 10 as shown in Fig. 13. Therefore, the valve housing 10 will move in relation to the socket 61, the outside surface of the housing which has been effectively covered by the socket so far being partially uncovered when the housing 10 is finally displaced to the position for locating the valve member 18 in the cut-out portion 29. It will accordingly be seen that the socket 61 forms an efficient protection against contamination of the exterior side surfaces of the valve 18.

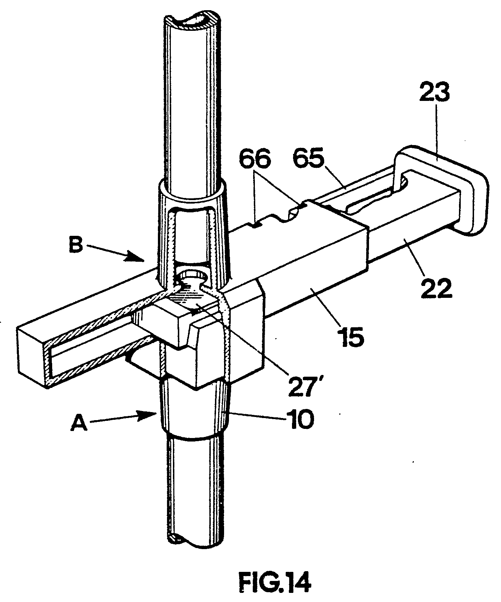

In Figs. 14 to 17 a further preferred embodiment of the slide valve and coupler assembly according to the present invention is shown.

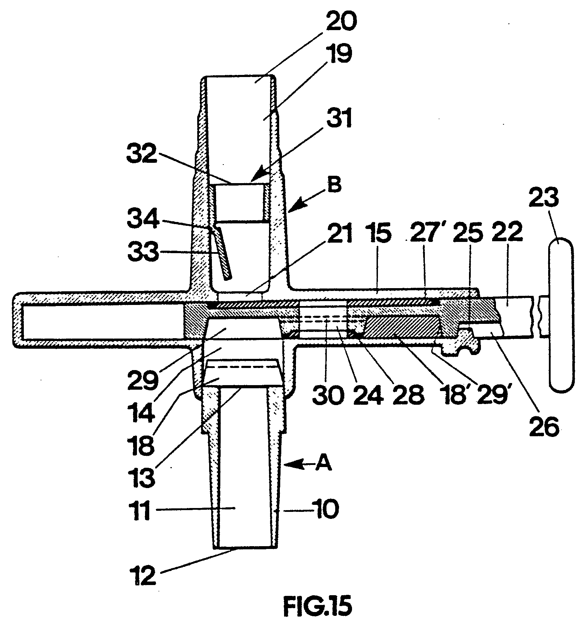

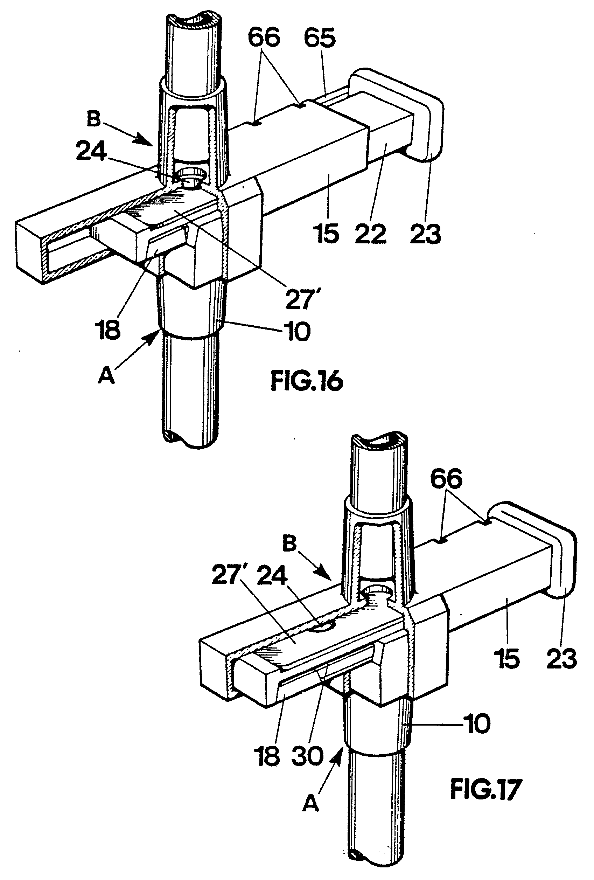

The operating slide 22' has a second cut-out portion 29' with a second valve member 18' on the opposite side of aperture 24. The gasket 27 of Figs. 2 to 4 is replaced by a sealing plate 27' of a resilient material such as rubber or plastics. The operating slide 22' can be displaced from a first position shown in Figs. 14 and 15, wherein the valve housing 10 is inserted in the recess 14, to a second position, shown in Fig. 16, wherein the passage through the assembly is established, and finally to a third position, shown in Fig. 17, wherein the second valve member 18' is displaced to and closes off the opening through the valve housing 10. In this third position the valve housing 10 can be removed from the coupler housing 15 with the slide valve member 18' connected therewith.

E.g. the slide valve and coupler assembly according to the invention can be used for peritoneal dialysis. In that case the slide valve is attached to the catheter leading to the abdominal cavity and is connected by means of the coupler to a plastics bag containing the sterilized solution, or to an apparatus for delivering such sterilized solution.

The embodiment of the slide valve and coupler assembly according to the present invention disclosed in Figs. 14 to 17 is used when there is a rigorous requirement for a sterile environment during connection and disconnection of the coupling members. The operating slide 22' and the coupler housing 15 accordingly are used only once to ensure the maintenance of said sterile environment. The slide valve A with valve member 18 and the coupler 8 with valve member 18' can be delivered enclosed within sterile housings or covers.

The slide valve and coupler assembly of the invention can be used with any desired receptacle or fluid supply. The operating slide is arrested in said three positions by means of a resilient arm 65 formed integrally with the finger rest and extending along the operating slide spaced therefrom. The arm engages by the inherent resiliency thereof one of three notches, corresponding one to each of the said three positions, which are provided in the coupler housing and two of which are shown at 66, but can be disengaged manually therefrom when it is desired to displace the operating slide.

Preferably the slide valve and coupler assembly is made of materials inert to the fluid to be carried, and non-toxic to the body. They can be of metal or plastics, e.g. aluminum, stainless steel, polypropylene, polyethylene, polyvinyl chloride, polyamide, polytetrafluoroethylene, polycarbonate polyoxymethylene, polyvinylidene chloride, natural and synthetic rubbers, polystyrene, and urea-formaldehyde and melamine-formaldehyde resins.

While not essential, if desired, a filter can be included in either of or both the slide valve housing and coupler housing, disposed across the fluid flow passage therethrough.

In order to further improve the safety against transfer of bacteria an absorbing material may be provided in the lower side of the operating slide 22 in a recess formed in the operating slide between the cut-out portion 29 and the through opening 24 so that a disinfection liquid absorbed by said material will wet the end surface of slide valve housing 10, when the valve member 18 is displaced from the closed position of Fig. 2 to the open position of Fig. 4.

In the embodiments described the operating means comprises a displaceable slide, but it is also possible to arrange such means as a rotatable body in the coupler housing 15.

## Claims

1. A slide valve and coupler assembly for controlling fluid flow, comprising a slide valve (A) including a valve housing (10) having a first through fluid flow passage (11), at least one slide valve member (18), and guide means (16, 17) in the housing for guiding the slide valve member along said guide means between a closed position across and closing off said first passage to fluid flow and an open position leaving the passage open for fluid flow, and a coupler (B) including a coupler housing (15) having a second through fluid flow passage (19) in alignment with the first passage at least when the valve is in one position, characterized in that said coupler comprises a first recess (14) for receiving at least the slide valve member-containing portion of the slide valve housing therein, operating means which is adjustable in said coupler housing between a first position across and closing off said second passage, and a second position leaving said second passage open for fluid flow, at least one second recess (29) in said operating means for receiving the slide valve member when the valve housing is inserted in the first recess, said operating means being adjustable for sliding the slide valve member to said closed position when the operating means is adjusted to said first position, and to said open position when the operating means is adjusted to said second position, and locking means (30) on said operating means connecting with the valve housing when the operating means is operated to said second position.

2. A slide valve and coupler assembly according to claim 1 wherein the valve housing (10) is shaped with a reentrant slot (16) serving as a track for the slide valve member (18), along which the valve member can be slid between said open and closed positions, and wherein the reentrant portion retains the valve member closely against the inner face of the housing with a sealing element or sealing action therebetween.

3. A slide valve and coupler assembly according to claim 1 or 2 wherein the operating means (22) comprises an operating slide displaceable or rotatable between said first and second positions.

4. A slide valve and coupler assembly according to claim 3 wherein the operating slide (22) has a through aperture (24) for connecting said first and second passages (11, 19) in said second position of the operating slide when the aperture is in register with the passages.

5. A slide valve and coupler assembly according to any of claims 1 to 4 wherein the slide valve (A) forms part of a receptacle or container (39, 40).

6. A slide valve and coupler assembly according to any of claims 1 to 5 wherein a check valve (31) is provided in said second flow passage (19) of the coupler (B).

7. A slide valve and coupler assembly according to claim 6 wherein means (54) are provided for positively operating the check valve (31) from the outside of the coupler housing (15).

8. A slide valve and coupler assembly according to claim 7 wherein said means for positively operating the check valve (31) comprises a tube (54) displaceable through the coupler housing (15) into said second passage (19).

9. A slide valve and coupler assembly according to any of claims 1 to 8 wherein disinfection means are provided in the wall of said first recess.

10. A slide valve and coupler assembly according to any of claims 1 to 9 wherein before insertion in the first recess the slide valve member (18) is surrounded by a flexible socket (61) connected to the slide valve housing (10) at a position spaced from the slide valve member, and wherein a shoulder (63) is provided in said first recess (14), the end of said socket engaging said shoulder when the slide valve member is inserted into said second recess (29).

## Patentansprüche

1. Absperrschieber- und Kupplungseinheit zur Steuerung eines Strömungsmittel-flusses, bestehend aus einem Absperrschieber (A) mit einem Schuibergehäuse (10) mit einer ersten Durchflußöffnung (11), wenigstens einem Ventilschieber (18) und einer Führungseinrichtung (16, 17) im Gehäuse zur Führung des Ventilschiebers entlang der Führungseinrichtung zwischen einer über der ersten Öffnung befindlichen und diese gegenüber dem Strömungsmittelfluß abschließenden Schließstellung und einer die Öffnung für den Strömungsmittelfluß freilassenden Offenstellung, und aus einer Kupplung (B) mit einem Kupplungsgehäuse (15) mit einer zweiten Durchflußöffnung (19), die zumindest bei in einer Stellung befindlichem Schieber mit der ersten Öffnung ausgerichtet ist, dadurch gekennzeichnet, daß die Kupplung eine erste Ausnehmung (14) zur Aufnahme zumindest des den Ventilschieber enthaltenden Teiles des Schiebergehäuses, eine Betätigungseinrichtung, die im Kupplungsgehäuse zwischen einer über der zweiten Öffnung befindlichen und diese verschließenden ersten Stellung und einer die zweite Öffnung für den Strömungsmittelfluß freilassenden zweiten Stellung verstellbar ist, wenigstens eine zweite Ausnehmung (29) in der Betätigungseinrichtung zur Aufnahme des Ventilschiebers bei in die erste Ausnehmung eingesetztem Schiebergehäuse, wobei die Betätigungseinrichtung zum Verschieben des Ventilschiebers in die Schließstellung bei in die erste Stellung eingestellter Betätigungseinrichtung und in die Offenstellung bei in die zweite Stellung eingestellter Betätigungseinrichtung verstellbar ist, und eine Sperreinrichtung (30) an der Betätigungseinrichtung aufweist, welche bei in die zweite Stellung verstellter Betätigungseinrichtung mit dem Schiebergehäuse verbunden ist.

2. Absperrschieber- und Kupplungseinheit nach Anspruch 1, dadurch gekennzeichnet, daß das Schiebergehäuse (10) mit einem hinterschnittenen Schlitz (16) ausgebildet ist, der als Bahn für den Ventilschieber (18) dient, entlang welcher der Ventilschieber zwischen der Offen- und der Schließstellung verschiebbar ist, und daß der Ventilschieber von dem hinterschnittenen Bereich eng an der Innenseite des Schiebergehäuses unter Zwischenlage eines Dichtelementes oder unter Dichtwirkung gehalten ist.

3. Absperrschieber- und Kupplungseinheit nach Anspruch 1 oder 2, dadurch gekennzeichnet, daß die Betätigungseinrichtung (22) aus einem Betätigungsschieber besteht, der zwischen der ersten und der zweiten Stellung verschiebbar oder verdrehbar ist.

4. Absperrschieber- und Kupplungseinheit nach Anspruch 3, dadurch gekennzeichnet, daß der Betätigungsschieber (22) eine Durchgangsöffnung (24) zur Verbindung der ersten und zweiten Durchflußöffnung (11, 19) in der zweiten Stellung des Betätigungsschiebers aufweist, in welcher die Durchgangsöffnung mit den Durchflußöffnungen fluchtet.

5. Absperrschieber- und Kupplungseinheit nach einem der Ansprüche 1 bis 4, dadurch gekennzeichnet, daß der Absperrschieber (A) einen Teil eines Gefäßes oder Behälters (39, 40) bildet.

6. Absperrschieber- und Kupplungseinheit nach einem der Ansprüche 1 bis 5, dadurch gekennzeichnet, daß in der zweiten Durchflußöffnung (19) der Kupplung (B) ein Rückschlagventil (31) vorgesehen ist.

7. Absperrschieber- und Kupplungseinheit nach Anspruch 6, dadurch gekennzeichnet, daß eine Einrichtung (54) zur zwangsweisen Betätigung des Rückschlagventiles (31) von der Außenseite des Kupplungsgehäuses (15) vorgesehen ist.

8. Absperrschieber- und Kupplungseinheit nach Anspruch 7, dadurch gekennzeichnet, daß die Einrichtung zur zwangsweisen Betätigung des Rückschlagventils (31) aus einem Rohr (54) besteht, das durch das Kupplungsgehäuse (15) in die zweite Durchflußöffnung (19) verschiebbar ist.

9. Absperrschieber- und Kupplungseinheit nach einem der Ansprüche 1 bis 8, dadurch gekennzeichnet, daß in der Wand der ersten Ausnehmung eine Desinfektionseinrichtung vorgesehen ist.

10. Absperrschieber- und Kupplungseinheit nach einem der Ansprüche 1 bis 9, dadurch gekennzeichnet, daß vor der Einführung in die erste Ausnehmung der Ventilschieber (18) von einer flexiblen Muffe (61) umgeben ist, die mit dem Schiebergehäuse (10) an einer im Abstand vom Ventilschieber befindlichen Stelle verbunden ist, und daß in der ersten Ausnehmung (14) ein Absatz (63) vorgesehen ist, wobei das Ende der Muffe bei in die zweite Ausnehmung (29) eingeführtem Ventilschieber an dem Absatz anschlägt.

**Revendications**

1. Dispositif de vanne à tiroir et d'accouplement pour le contrôle d'un courant de fluide, comprenant une vanne à tiroir (A) comportant une enveloppe (10) présentant un premier canal traversant (11) pour le passage d'un fluide, au moins un élément (18) de vanne à tiroir, et un moyen de guidage (16, 17) dans l'enveloppe pour guider l'élément de vanne à tiroir le long du moyen de guidage entre une position formée où il est à travers le premier canal pour le fermer au passage du courant de fluide et une position ouverte laissant le canal ouvert au passage du courant de fluide, et un coupleur (B) comportant une enveloppe (15) munie d'un second canal traversant de passage de fluide (19) aligné avec le premier canal au moins lorsque la vanne se trouve dans l'une des positions, caractérisé en ce que le coupleur comporte un premier évidement (14) destiné à recevoir au moins la partie contenant l'élément de vanne à tiroir de l'enveloppe de la vanne à tiroir, un moyen d'actionnement qui est réglable dans l'enveloppe du coupleur entre une première position où il est à travers le second canal et le ferme, et une seconde position laissant ce second canal ouvert au passage du courant de fluide, au moins un second évidement (29) dans le moyen d'actionnement pour recevoir l'élément de vanne à tiroir lorsque le logement de vanne est inséré dans le premier évidement, ce moyen d'actionnement étant réglable pour faire coulisser l'élément de vanne à tiroir vers la position fermée lorsque le moyen d'actionnement est réglé à la première position, et vers la position ouverte lorsque le moyen d'actionnement est réglé à la seconde position, et un moyen de blocage (30) sur le moyen d'actionnement en liaison avec l'enveloppe de vanne lorsque ce moyen d'actionnement est amené à la seconde position.

2. Dispositif de vanne à tiroir et d'accouplement selon la revendication 1, caractérisé en ce que l'enveloppe de vanne (10) est munie d'une rainure ré-entrante (16) servant de trajet pour l'élément de vanne à tiroir (18), le long de laquelle l'élément de vanne peut coulisser entre les positions ouverte et fermée, et en ce que la partie ré-entrante maintient étroitement l'élément de vanne contre la face intérieure de l'enveloppe avec un élément d'étanchéité ou effet d'étanchétté entre eux.

3. Dispositif de vanne à tiroir et d'accouplement selon la revendication 1 ou 2, caractérisé en ce que le moyen d'actionnement (22) comprend une coulisse opérationnelle pouvant être déplacée ou mise en rotation entre les première et seconde positions.

4. Dispositif de vanne à tiroir et d'accouplement selon la revendication 3, caractérisé en ce que la coulisse opérationnelle (22) comporte une ouverture traversant (24) pour relier les premier et second canaux (11, 19) dans la seconde position de la coulisse lorsque l'ouverture coïncide avec les canaux.

5. Dispositif de vanne à tiroir et d'accouplement selon l'une quelconque des revendications 1 à 4, caractérisé en ce que la vanne à tiroir (A) constitue une partie d'un récipient ou conteneur (39, 40).

6. Dispositif de vanne à tiroir et d'accouplement selon l'une quelconque des revendications 1 à 5, caractérisé en ce qu'une soupape d'arrêt (31) est prévue dans le second canal (19) du coupleur (B).

7. Dispositif de vanne à tiroir et d'accouplement selon la revendication 6, caractérisé en ce qu'un moyen (54) est prévu pour actionner positivement la soupape d'arrêt (31) à partir de l'extérieur de l'enveloppe de coupleur (15).

8. Dispositif de vanne à tiroir et d'accouplement selon la revendication 7, caractérisé en ce que le moyen permettant d'actionner positivement la soupape d'arrêt (31) comprend un tube (54) pouvant être déplacé par l'intermédiaire de l'enveloppe de coupleur (15) dans le second canal (19).

9. Dispositif de vanne à tiroir et d'accouplement selon l'une quelconque des revendications 1 à 8, caractérisé en ce qu'un moyen de désinfection est prévu dans la paroi du premier évidement.

10. Dispositif de vanne à tiroir et d'accouplement selon l'une quelconque des revendications 1 à 9, caractérisé en ce que, avant insertion dans le premier évidement, l'élément de vanne à tiroir (18) est entouré par une douille flexible (61) reliée à l'enveloppe de vanne à tiroir (10) en un endroit espacé de l'élément de vanne à tiroir, et en ce qu'un épaulement (63) est prévu dans le premier évidement (15), l'extrémité de la douille venant en contact avec l'épaulement lorsque l'élément de vanne à tiroir est inséré dans le second évidement (29).

FIG.1

FIG.2

FIG. 3

FIG.4

FIG.5

FIG.6

FIG.7

FIG.9

FIG.8

FIG.10

FIG.11

FIG.12

FIG.13

FIG.14

FIG.15

FIG.16

FIG.17